# EUROPEAN PATENT APPLICATION

(11) **EP 1 506 956 A1**
(43) Date of publication of application: **16.02.2005**
(21) Application number: 03728058.3
(22) Date of filing: 13.05.2003
(51) Int. Cl.: C07C 251/22, C07C 249/02, C07C 253/30, C07C 255/42, C09K 3/00, G02B 5/22

(54) **DIIMONIUM SALT MIXTURES, AMINIUM SALTS MIXTURES AND USE THEREOF**

(30) Priority: 20.05.2002 JP 2002144263; 21.06.2002 JP 2002180760; 08.07.2002 JP 2002198234; 30.07.2002 JP 2002221358
(71) Applicant: Nippon Kayaku Kabushiki Kaisha, Tokyo 102-8172 (JP)
(72) Inventor: KITAYAMA, Yasuyuki, Hartsdale, NY 10530 (US); YAMAMURA, Shigeo, Saitama-shi, Saitama 331-0812 (JP)
(74) Representative: Gille Hrabal Struck Neidlein Prop Roos
(86) International application number: PCT/JP2003/005930
(87) International publication number: WO 2003/097580

(57) **Abstract**

A mixture of two or more diimonium salts or aminium salts obtained by oxidizing one or more compounds represented by the general formula (1); near-infrared absorption filters characterized by containing the mixture; and optical information recording media characterized by containing the mixture in the recording layer: (1) 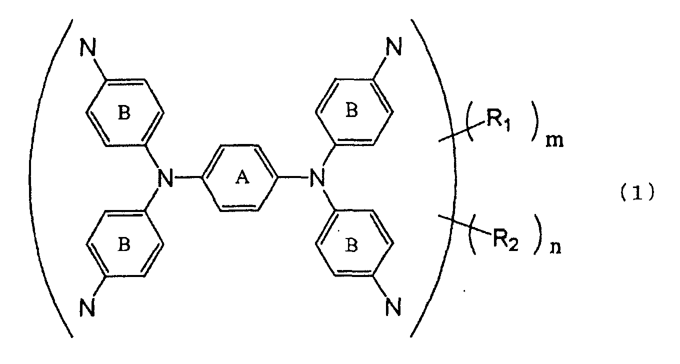 wherein A and B may have additional substituents; R₁ and R₂ attached to terminal nitrogen atoms of tetrakis(aminophenyl)-phenylenediamine skeleton are different from each other and each substituted or unsubstituted alkyl having 1 to 8 carbon atoms, cycloalkyl, alkenyl, or aryl; and m and n are integers of 0 to 8 satisfying the relationship: m+n = 8.

## Description

### Technical Field

The present invention relates to a diimonium salt compound and an aminium salt compound having an absorption in an infrared region, in particular, a diimonium salt compound and an aminium salt compound excellent in heat resistance and moisture heat resistance, and high in solubility. The present invention also relates to a near-infrared absorption filter and a near-infrared absorption composition containing a mixture of the compounds, which are excellent in heat resistance, and an optical information recording medium excellent in lightfastness.

### Background Art

Diimonium salt compounds and aminium salt compounds, which are near-infrared absorbents, have been widely used for a near-infrared absorption filter, a heat insulating film and sunglasses. However, degradation easily occurs due to a heat treatment during production, and similar degradation such as change of hue also occurs upon environmental tests such as a heat resistance test and a moisture heat resistance test, posing a problem of heat resistance and moisture heat resistance of a dye itself.

Conventional near-infrared absorption filters have had a problem of reduced near-infrared absorbability, because they can be easily denatured by heat or light. In particular, a serious problem has been caused by heat that a visible light transmittance of the filter itself is reduced due to decomposition and a hue thereof becomes greenish.

On the other hand, as a dye used for an optical information recording medium containing an organic dye, in particular an optical disk such as CD-R and DVD-R and an optical card which can record only once, various kinds of organic dyes such as a cyanine dye have been proposed. Generally, however, such organic dye is easily denatured by light and this has brought about a problem that the record reproduction property and the storage stability are degraded.

As means for solving these problems, in Japanese Patent Application Laying Open (KOKAI) No. 2000-81511, a dye containing a cyanoalkyl group at a terminal nitrogen atom of a tetrakis(aminophenyl)phenylenediamine skeleton is proposed. While the heat resistance and light resistance thereof have been improved, there has been a problem that the dye becomes insoluble in solvents generally used. Regarding solubility, a certain improvement effect can be found by changing counter ions, but it is not satisfactory. Further, depending on the property of the counter ions, even the original heat resistance and light resistance may be damaged. As a method for further improving solubility, controlling the number of substituents to be introduced to the eight bonding sites of the terminal nitrogen atoms, for example, to introduce four cyanoalkyl groups and substitute the rest four with alkyl groups to synthesize a compound is also proposed as described in the above-mentioned publication. However, to achieve this, a special reaction step of protecting the reaction site or purification during reaction is necessary. Moreover, control of the reaction is difficult due to the change in reactivity under the influence of the temperature and the equivalent amount of reagent, and isolation has often been extremely difficult even if synthesis has been successful. Therefore, an optical information recording medium containing the dye as proposed has been impractical.

### Problems to be Solved by the Invention

The present invention has been made in view of such circumstances. An object of the present invention is to provide a mixture of diimonium salt compounds and a mixture of aminium salt compounds that can be synthesized with ease as a mixture, which is superior in heat resistance and moisture heat resistance and has a higher solubility than that of conventional ones. Another object of the present invention is to provide a near-infrared absorption filter suitable for a plasma display panel, which has improved heat resistance, moisture heat resistance and light resistance, using a near-infrared absorption compound of which stability, in particular, the heat resistance, moisture heat resistance and light resistance are improved compared to the conventional diimonium salts. Another object of the present invention is to provide an optical information recording medium which is particularly excellent in light resistance and has repetition durability, as compared with conventional optical information recording media containing an aminium salt and a diimonium salt, by using a dye having such a solubility that does not affect processability.

### Disclosure of the Invention

The present inventors have conducted intensive studies in an attempt to solve the above-mentioned problems and found that a mixture of oxides obtained by oxidizing compounds having a structure represented by the formula (1), which is a mixture of diimonium salts and a mixture of aminium salts, is excellent in heat resistance and moisture heat resistance and high in solubility, which resulted in the completion of the present invention. Accordingly, the present invention relates to
(1) A mixture comprising two or more oxides obtained by oxidizing two or more compounds represented by the formula (1): 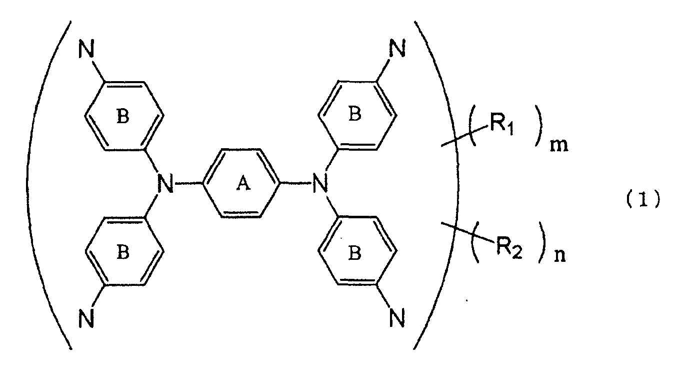 wherein ring A and ring B optionally have substituents, R₁ and R₂ attached to terminal nitrogen atoms of a tetrakis(aminophenyl)-phenylenediamine skeleton are different from each other and each being a substituted or unsubstituted alkyl group having 1 to 8 carbon atoms, a cycloalkyl group, an alkenyl group or an aryl group, and m and n are integers of 0 to 8 satisfying the relationship: m+n=8;
(2) a mixture comprising two or more diimonium salt compounds obtained by oxidizing two or more compounds represented by the formula (1): 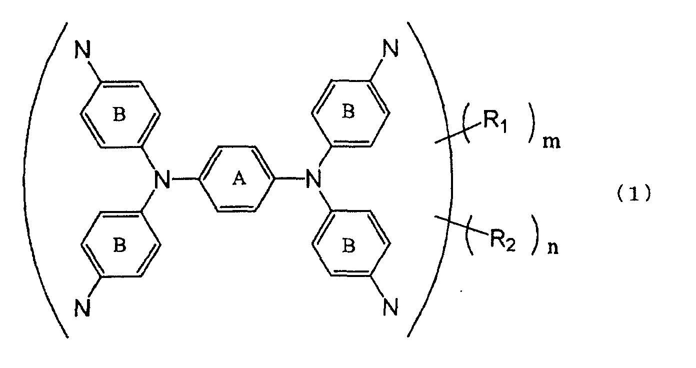 wherein ring A and ring B optionally have substituents, R₁ and R₂ attached to terminal nitrogen atoms of a tetrakis(aminophenyl)-phenylenediamine skeleton are different from each other and each being a substituted or unsubstituted alkyl group having 1 to 8 carbon atoms, a cycloalkyl group, an alkenyl group or an aryl group, and m and n are integers of 0 to 8 satisfying the relationship: m+n=8;
(3) the mixture of diimonium salt compounds of (2), wherein the ring A and the ring B have no substituent or have, as substituents, at least one selected from the group consisting of a halogen atom(s), an alkyl group (s) and a cyano group (s) ;
(4) the mixture of diimonium salt compounds of (3), wherein R₁ and R₂ are a substituted or unsubstituted alkyl group (s) or an alkenyl group(s);
(5) the mixture of diimonium salt compounds of (4), wherein at least one of R₁ and R₂ is a cyano(C1-C6)alkyl group;
(6) the mixture of diimonium salt compounds of (5), wherein the cyano(C1-C6)alkyl group is a cyanopropyl group;
(7) a near-infrared absorption filter comprising the mixture of diimonium salt compounds of any one of (2) to (6);
(8) a near-infrared absorption composition obtained by adding the mixture of diimonium salt compounds of any one of (2) to (6) to a resin(s);
(9) an optical information recording medium containing, in a recording layer, the mixture of diimonium salt compounds of any one of (2) to (6);
(10) a mixture comprising two or more aminium salt compounds obtained by oxidizing two or more compounds represented by the formula (1): 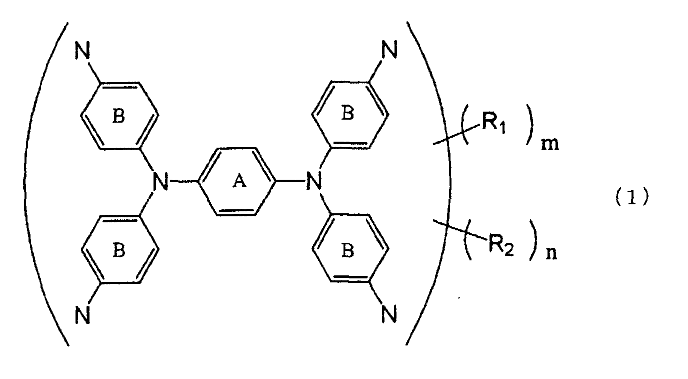 wherein ring A and ring B optionally have substituents, R₁ and R₂ attached to terminal nitrogen atoms of a tetrakis(aminophenyl)phenylenediamine skeleton are different from each other and each being a substituted or unsubstituted alkyl group having 1 to 8 carbon atoms, a cycloalkyl group, an alkenyl group or an aryl group, and m and n are integers of 0 to 8 satisfying the relationship: m+n=8;
(11) the mixture of aminium salt compounds of (10), wherein the ring A and the ring B have no substituent or have, as substituents, at least one selected from the group consisting of a halogen atom(s), an alkyl group (s) and a cyano group(s);
(12) the mixture of aminium salt compounds of (11), wherein R₁ and R₂ are a substituted or unsubstituted alkyl group(s) or an alkenyl group(s);
(13) the mixture of aminium salt compounds of (12), wherein at least one of R₁ and R₂ is a cyano(C1-C6)alkyl group;
(14) the mixture of aminium salt compounds of (13), wherein the cyano(C1-C6)alkyl group is a cyanopropyl group;
(15) a near-infrared absorption filter comprising the mixture of aminium salt compounds of any one of (10) to (14);
(16) a near-infrared absorption composition obtained by adding the mixture of aminium salt compounds of any one of (10) to (14) to a resin(s);
(17) an optical information recording medium containing, in a recording layer, the mixture of aminium salt compounds of any one of (10) to (14);
(18) a mixture of two or more oxide precursors which can produce the mixture of diimonium salt compounds of any one of (2) to (6) or the mixture of aminium salt compounds of any one of (10) to (14);
(19) a mixture of diimonium salt compounds containing two or more compounds represented by the formula (2): 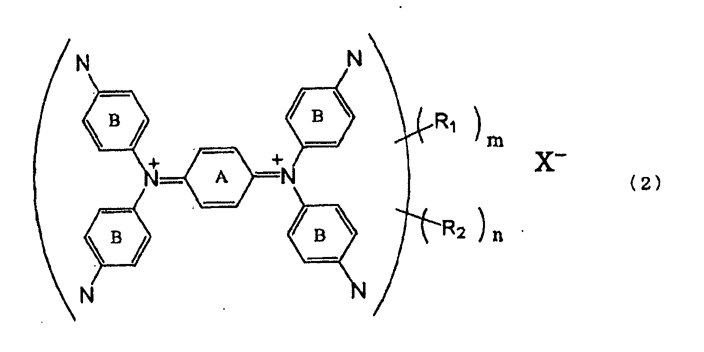 wherein ring A and ring B optionally have substituents, R₁ and R₂ attached to terminal nitrogen atoms of a tetrakis(aminophenyl)phenylenediamine skeleton are different from each other and each being a substituted or unsubstituted alkyl group having 1 to 8 carbon atoms, a cycloalkyl group, an alkenyl group or an aryl group, m and n are integers of 0 to 8 satisfying the relationship: m+n=8 and X is an atomic group having a negative electric charge(s) necessary for neutralizing a positive electric charge(s); and
(20) a mixture of aminium salt compounds containing two or more compounds represented by the formula (3): 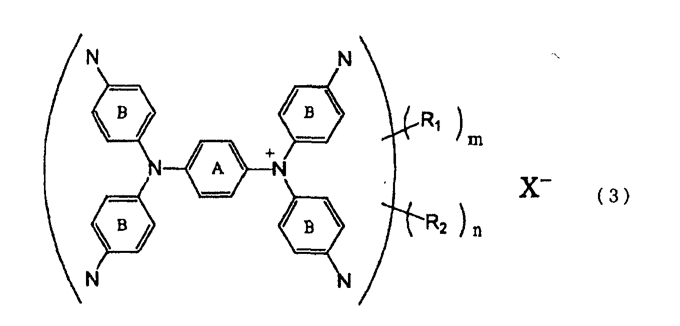
wherein ring A and ring B optionally have substituents, R₁ and R₂ attached to terminal nitrogen atoms of a tetrakis(aminophenyl)phenylenediamine skeleton are different from each other and each being a substituted or unsubstituted alkyl group having 1 to 8 carbon atoms, a cycloalkyl group, an alkenyl group or an aryl group, m and n are integers of 0 to 8 satisfying the relationship: m+n=8 and X is an atomic group having a negative electric charge(s) necessary for neutralizing a positive electric charge(s).

### Best Mode for Carrying Out the Invention

### Mixture of diimonium salt compounds

The mixture of diimonium salt compounds of the present invention can be obtained by oxidizing two or more compounds having a structure represented by the formula (1). A typical example of the mixture of such diimonium salt compounds can be represented by the following formula (2): 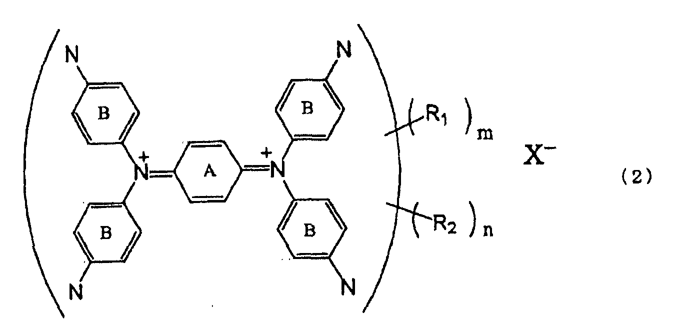 wherein ring A, ring B, R₁, R₂, m and n are as defined above and X is an atomic group having a negative electric charge (s) necessary for neutralizing a positive electric charge(s).

In the formula (2), ring A and ring B may further have 1 to 4 substituents, R₁ and R₂ attached to terminal nitrogen atoms of a tetrakis(aminophenyl)phenylenediamine skeleton are different from each other and each being a substituted or unsubstituted alkyl group having 1 to 8 carbon atoms, a cycloalkyl group, an alkenyl group or an aryl group. X is an atomic group having a negative electric charge (s) necessary for neutralizing a positive electric charge(s) and m and n are integers of 0 to 8 satisfying the relationship: m+n=8.

Ring A may or may not have 1 to 4 substituents. Examples of the substituent that can be bonded include a halogen atom, a hydroxyl group, an alkoxy group, a cyano group and an alkyl group. Examples of the halogen atom include a fluorine atom, a chlorine atom, a bromine atom and an iodine atom. Examples of the alkoxy group include C₁-C₅ alkoxy groups such as a methoxy group and an ethoxy group, and examples of the alkyl group include C₁-C₅ alkyl groups such as a methyl group and an ethyl group. It is preferable that A does not have a substituent or is substituted by a halogen atom (in particular, a chlorine atom and a bromine atom), a methyl group or a cyano group.

Examples of the substituent capable of substitution for ring B other than an amino group as described above include a halogen atom, a hydroxyl group, an alkoxy group, a cyano group and an alkyl group. Examples of the halogen atom include a fluorine atom, a chlorine atom, a bromine atom and an iodine atom. Examples of the alkoxy group include C1-C5 alkoxy groups such as a methoxy group and an ethoxy group, and examples of the alkyl group include C1-C5 alkyl groups such as a methyl group and an ethyl group.

Examples of the alkyl group at R₁ and R₂ are, for example, a methyl group, an ethyl group, a propyl group, a butyl group and a pentyl group. The alkyl moietymaybe a linear or branched chain and may have substituents. Examples of the substituent that can be bonded include a halogen atom (e.g., F, Cl, Br), a hydroxyl group, an alkoxy group ( e . g . , methoxy group, ethoxy group, isobuthoxy group), an alkoxyalkoxy group (e.g., methoxyethoxy group), an aryl group (e.g., phenyl group, naphthyl group), an aryloxy group (e.g., phenoxy group), an acyloxy group (e.g., acetyloxy group, butyryloxy group, hexyryloxy group, benzoyloxy group), an alkyl substituted amino group (e.g., methylamino group, dimethylamino group), a cyano group, a nitro group, a carboxyl group and a sulfo group.

Examples of the cycloalkyl group include a cyclopentyl group and a cyclohexyl group. Examples of the alkenyl group include an allyl group, a 1-butenyl group and a 1-pentenyl group. Examples of the aryl group include a phenyl group and a naphthyl group, and the aryl group may have substituents. Examples of the substituent include an alkyl group having 1 to 8 carbon atoms (e.g., methyl group, ethyl group, butyl group), an alkoxy group having 1 to 6 carbon atoms (e.g., methoxy group, ethoxy group), an aryloxy group (e. g. , phenoxy group, p-chlorophenoxy group), a halogen atom (e.g., F, Cl, Br), alkoxycarbonyl group (e.g., methoxycarbonyl group, ethoxycarbonyl group), an amino group, alkyl substituted amino group (e.g., methylamino group), an amide group (e. g. , acetoamide group), sulfonamide group (e.g., methane sulfoneamide group) , a cyano group, a nitro group, a carboxyl group and a sulfo group. These aryl groups preferably have 6 to 12 carbon atoms.

A particularly preferable substituent for R₁ and R₂ is an unsubstituted alkyl group, a cyano-substituted alkyl group, an alkoxy-substituted alkyl group and an allyl group. Examples of the unsubstituted alkyl group include (C1-C8) alkyl groups such as a methyl group, an ethyl group, an n-propyl group, an isopropyl group, an n-butyl group, a sec-butyl group, a tert-butyl group, a pentyl group, a hexyl group and a heptyl group. Examples of the cyano-substituted alkyl group include cyano-substituted (C1-C6) alkyl groups such as a cyanomethyl group, 2-cyanoethyl group, 3-cyanopropyl group, 2-cyanopropyl group, 4-cyanobutyl group, 3-cyanobutyl group, 2-cyanobutyl group, 5-cyanopentyl group, 4-cyanopentyl group, 3-cyanopentyl group and 2-cyanopentyl group. Examples of the alkoxy-substituted alkyl group include alkoxy-substituted(C1-C6)alkyl groups such as methoxyethyl group, ethoxyethyl group, 3-methoxypropyl group, 3-ethoxypropyl group, 4-methoxybutyl group, 4-ethoxybutyl group, 5-ethoxypentyl group and 5-methoxypentyl group.

In consideration of the heat resistance and moisture heat resistance, either R₁ or R₂ is more preferably a cyano-substituted(C1-C6)alkyl group. Examples of the cyano-substituted(C1-C6)alkyl group are as mentioned above, and more preferably a cyanopropyl group.

m and n indicate the number of substituents introduced into R₁ and R₂, which are integers of 0-8 satisfying the relationship: m+n=8.

In the formula (2) , X is an atomic group having a negative electric charge(s) necessary for neutralizing a positive electric charge(s). As examples thereof, a monovalent anion such as monovalent organic acid anions and monovalent inorganic acid anions can be mentioned. Examples of the monovalent organic acid anions include organic carboxylate ions such as an acetate ion, a lactate ion, a trifluoroacetate ion, a propionate ion, a benzoate ion, an oxalate ion, a succinate ion and a stearate ion; organic sulfonate ions such as a methanesulfonate ion, a toluenesulfonate ion, a naphthalenemonosulfonate ion, a chlorobenzesulfonate ion, a nitrobenzenesulfonate ion, a dodecylbenzenesulfonate ion, a benzenesulfonate ion, an ethanesulfonate ion and a trifluoromethanesulfonate ion; and organic borate ions such as a tetraphenylborate ion and a butyltriphenylborate, and halogenoalkylsulfonate ions such as a trifluoromethanesulfonate ion and a toluenesulfonate ion or alkylarylsulfonate ions are preferable.

Examples of the monovalent inorganic anion include halogen ions such as a fluorine ion, a chlorine ion, a bromine ion and an iodine ion; a thiocyanate ion, a hexafluoroantimonate ion, a perchlorate ion, a periodate ion, a nitrate ion, a tetrafluoroborate ion, a hexafluorophosphate ion, a molybdate ion, a tungstate ion, a titanate ion, a vanadate ion, a phosphate ion, a borate ion and the like, and a perchlorate ion, an iodine ion, a tetrafluoroborate ion, a hexafluorophosphate ion and a hexafluoroantimanate ion are preferable.

Examples of the divalent anion include naphthalene disulfonic acid derivatives such as naphthalene-1,5-disulfonic acid, R acid, G acid, H acid, benzoyl H acid, p-chlorobenzoyl H acid, p-toluenesulfonyl H acid, chloro H acid, chloroacetyl H acid, metanilic γ acid, 6-sulfonaphthyl-γ acid, C acid, ε acid, p-toluenesulfonyl R acid, naphthalene-1,6-disulfonic acid and 1-naphthol-4,8-disulfonic acid, divalent organic acid ions such as carbonyl J acid, 4,4'-diaminostilbene-2,2'-disulfonic acid, di J acid, naphthalic acid, naphthalene-2,3-dicarboxylic acid, diphenic acid, stilbene-4,4'-dicarboxylic acid, 6-sulfo-2-oxy-3-naphthoic acid, anthraquinone-1,8-disulfonic acid, 1,6-diaminoanthraquinone-2,7-disulfonic acid, 2-(4-sulfophenyl)-6-aminobenzotriazole-5-sulfonic acid, 6-(3-methyl-5-pyrazolonyl)-naphthalene-1,3-disulfonic acid and 1-naphthol-6-(4-amino-3-sulfo)anilino-3-sulfonic acid.

In the diimonium salt compound, when the anion is monovalent, two molecules are necessary. When the anion is divalent as in 1, 5-naphthalene disulfonic acid, one molecule is necessary.

Of these atomic groups having a negative electric charge(s), a perchlorate ion, an iodine ion, a tetrafluoroborate ion, a hexafluorophosphate ion, a hexafluoroantimonate ion, trifluoromethanesulfonate ion and toluenesulfonate ion are preferable.

Specific examples of the diimonium salt compound of the present invention represented by the formula (2) are shown in Tables 1-3. In Tables 1-3, 1,5-NpS represents 1, 5-naphthalene disulfonic acid and TsO represents a toluene sulfonate ion. Unsubstituted A is simply described as "4H" and the substituted position in A is described as ortho (o-) or meta (m-) with one of the nitrogen atoms of the phenylenediamine skeleton as a center. The substituted position in B is described as 1-4 clockwise relative to the nitrogen atom that bonds to the phenylenediamine skeleton. In addition, a compound in which m=1, n=7 is described as (1,7) and a mixture comprising a compound in which m=1, n=7 and a compound in which m=2, n=6 is simply described as (1,7)(2,6).

**TABLE 1**

| No. | R ₁ | R ₂ | A | B | X | (m, n) |
|---|---|---|---|---|---|---|
| 1a | n-C4H9 | C3H6CN | 4H | 4H | SbF6 | (4,4)(3,5)(2,6)(1,7) |
| 2a | n-C4H9 | C3H6CN | 4H | 4H | PF6 | (4,4)(3,5)(2,6)(1,7) |
| 3a | n-C4H9 | C3H6CN | 4H | 4H | PF6 | (5,3)(4,4)(3,5)(2,6) (1,7) |
| 4a | n-C4H9 | C3H6CN | 4H | 4H | BF4 | (4,4)(3,5)(2,6)(1,7) |
| 5a | n-C4H9 | C3H6CN | 4H | 4H | ClO4 | (2,6)(1,7) |
| 6a | i-C5H11 | C3H6CN | 4H | 4H | SbF6 | (7,1)(6,2)(5,3)(4,4) |
| 7a | i-C5H11 | C3H6CN | 4H | 4H | PF6 | (7,1)(6,2)(5,3)(4,4) |
| 8a | i-C5H11 | C3H6CN | 4H | 4H | BF4 | (7,1)(2,6) |
| 9a | i-C5H11 | C3H6CN | 4H | 4H | ClO4 | (7,1)(6,2) |
| 10a | i-C4H9 | n-C4H9 | 4H | 4H | PF6 | (5,3)(4,4)(3,5) |
| 11a | i-C4H9 | n-C4H9 | 4H | 4H | SbF6 | (7,1)(6,2) |
| 12a | C2H40CH3 | C3H6CN | 4H | 4H | PF6 | (4,4)(3,5)(2,6)(1,7) |
| 13a | C2H40CH3 | C3H6CN | 4H | 4H | SbF6 | (4,4)(3,5)(2,6)(1,7) |

**TABLE 2**

| No. | R ₁ | R ₂ | A | B | X | (m, n) |
|---|---|---|---|---|---|---|
| 14a | i-C4H9 | C3H6CN | 4H | 4H | SbF6 | (7,1)(6,2)(5,3) |
| 15a | i-C5H11 | i-C4H9 | 4H | 4H | SbF6 | (5,3)(4,4)(3,5) |
| 16a | i-C4H9 | C3H6CN | 4H | 4H | PF6 | (4,4)(3,5)(2,6)(1,7) |
| 17a | CH2CH=CH2 | C3H6CN | 4H | 4H | 1,5-NpS | (7,1)(6,2) |
| 18a | CH2CH=CH2 | C3H6CN | 4H | 4H | PF6 | (4,4)(3,5)(2,6)(1,7) |
| 19a | i-C5H11 | i-C4H9 | 4H | 4H | PF6 | (7,1)(6,2)(5,3)(4,4) |
| 20a | i-C5H11 | n-C4H9 | 4H | 4H | SbF6 | (5,3)(4,4)(3,5) |
| 21a | C2H40CH3 | n-C4H9 | 4H | 4H | PF6 | (4,4)(3,5)(2,6)(1,7) |
| 22a | n-C4H9 | C3H6CN | o-Cl | 4H | PF6 | (7,1)(6,2)(5,3) |
| 23a | n-C4H9 | C3H6CN | m-CH3 | 4H | BF4 | (4,4)(3,5)(2,6)(1,7) |
| 24a | i-C5H11 | C3H6CN | 4H | 2-Cl | ClO4 | (7,1)(6,2) |
| 25a | i-C5H11 | C3H6CN | 4H | 2-CH3 | SbF6 | (7,1)(6,2)(5,3) |

**TABLE 3**

| No. | R ₁ | R ₂ | A | B | X | (m, n) |
|---|---|---|---|---|---|---|
| 26a | Cy-C6H12 | C3H6CN | 4H | 4H | SbF6 | (7,1)(6,2)(5,3) |
| 27a | CH2Ph | n-C4H9 | 4H | 4H | TsO | (4,4)(3,5)(2,6)(1,7) |
| 28a | Ph | n-C4H9 | o-Cl | 4H | ClO4 | (2,6)(1,7) |
| 29a | i-C5H11 | C4H8CN | 4H | 4H | SbF6 | (5,3)(4,4)(3,5) |
| 30a | s-C5H11 | C2H40CH3 | 4H | 4H | PF6 | (2,6)(1,7) |
| 31a | n-C5H11 | C3H6CN | 4H | 4H | PF6 | (8,0)(7,1)(6,2)(5,3) |
| 32a | n-C5H11 | C3H6CN | 4H | 4H | SbF6 | (5,3)(4,4)(3,5) |
| 33a | n-C2H5 | i-C5H11 | 4H | 4H | PF6 | (3,5)(2,6)(1,7)(0,8) |
| 34a | C2H4OPh | n-C4H9 | 4H | 4H | PF6 | (7,1)(6,2) |
| 35a | CH2CH=CH2 | i-C5H11 | 4H | 4H | BF4 | (4,4)(3,5)(2,6)(1,7) |
| 36a | i-C5H11 | C2H40CH3 | 4H | 4H | ClO4 | (4,4)(3,5)(2,6)(1,7) |
| 37a | i-C5H11 | C3H6CN | o-Cl | 4H | SbF6 | (7,1)(6,2)(5,3) |

The mixture of diimonium salt compounds represented by the formula (2) of the present invention can be obtained by the following method described, for example, in Japanese Patent Publication (KOKOKU) No. 43-25335. That is, an amino compound represented by the following formula (4) 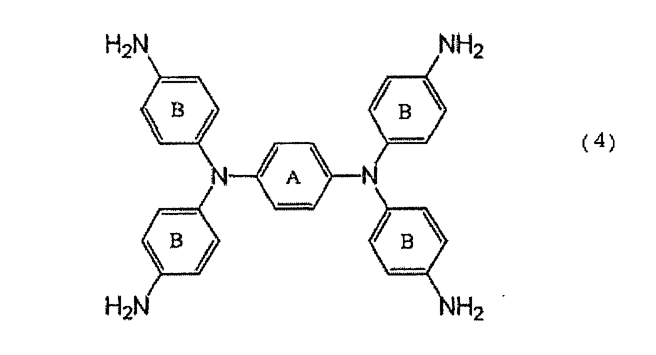 wherein ring A and B are as defined above, obtained by a Ullmann reaction and reduction reaction, is mixed, in an organic solvent, preferably an aqueous polar solvent such as DMF, DMI or NMP, at 30 to 160°C, preferably 50 to 140°C, with a halogenated compound corresponding to R₁ (e.g., BrC4H9 when R₁ is n-C4H9) and a halogenated compound corresponding to R₂ (e. g. , BrCH2CH (CH3) 2 when R₂ is i-C4H9) in any proportion, and reacted to obtain a mixture of compounds represented by the formula (1). To control the ratio of m to n, it is also possible to synthesize a mixture of compounds in any proportion by reacting the halogenated compound corresponding to R₁ in advance and then reacting the halogenated compound corresponding to R₂.

Then the mixture of the compounds synthesized above is subjected to oxidization reaction by adding 2 equivalents of an oxidant (e.g., silver salt) corresponding to X of the formula (2) at 0 to 100°C, preferably 5 to 70°C, using an organic solvent, preferably an aqueous polar solvent such as DMF, DMI or NMP, to obtain a mixture of diimonium salt compounds represented by the formula (2) of the present invention.

### Mixture of aminium salt compounds

The mixture of aminium salt compounds of the present invention can be obtained by oxidizing two or more compounds having a structure represented by the formula (1). A typical example of the mixture of such aminium salt compounds can be represented by the following formula (3): 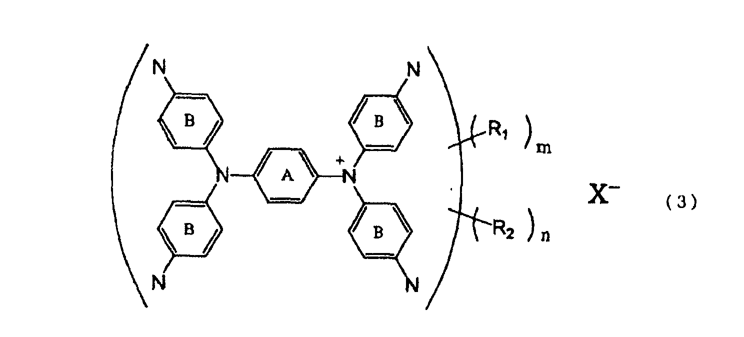 wherein ring A, ring B, R₁, R₂, m and n are as defined above and X is an atomic group having a negative electric charge (s) necessary for neutralizing a positive electric charge(s).

In the formula (3), ring A and ring B may further have 1 to 4 substituents, R₁ and R₂ attached to terminal nitrogen atoms of a tetrakis(aminophenyl)-phenylenediamine skeleton are different from each other and each being a substituted or unsubstituted alkyl group having 1 to 8 carbon atoms, a cycloalkyl group, an alkenyl group or an aryl group. X is an atomic group having a negative electric charge (s) necessary for neutralizing a positive electric charge(s) and m and n are integers of 0 to 8 satisfying the relationship: m+n=8.

Ring A may or may not have 1 to 4 substituents. Examples of the substituent that can be bonded include a halogen atom, a hydroxyl group, an alkoxy group, a cyano group and an alkyl group. Examples of the halogen atom include a fluorine atom, a chlorine atom, a bromine atom and an iodine atom. Examples of the alkoxy group include C1-C5 alkoxy groups such as a methoxy group and an ethoxy group and examples of the alkyl group include C1-C5 alkyl groups such as a methyl group and an ethyl group. It is preferable that A does not have a substituent or is substituted with a halogen atom (in particular, a chlorine atom and a bromine atom), a methyl group or a cyano group.

Examples of the substituent capable of substitution for ring B other than an amino group as described above, include a halogen atom, a hydroxyl group, an alkoxy group, a cyano group and an alkyl group. Examples of the halogen atom include a fluorine atom, a chlorine atom, a bromine atom and an iodine atom. Examples of the alkoxy group include C1-C5 alkoxy groups such as a methoxy group and an ethoxy group and examples of the alkyl group include C1-C5 alkyl groups such as a methyl group and an ethyl group.

The alkyl group at R₁ and R₂ includes, for example, a methyl group, an ethyl group, a propyl group, a butyl group and a pentyl group. The alkyl moiety may be in a linear or branched chain and may have substituents. Examples of the substituent that can be bonded include a halogen atom (e.g., F, Cl, Br), a hydroxyl group, an alkoxy group (e.g., methoxy group, ethoxy group, isobuthoxy group), an alkoxyalkoxy group (e. g. , methoxyethoxy group), an aryl group (e. g. , phenyl group, naphthyl group), an aryloxy group (e.g., phenoxy group), an acyloxy group (e.g., acetyloxy group, butyryloxy group, hexyloxy group, benzoyloxy group), an alkyl substituted amino group (e.g., methylamino group, dimethylamino group),a cyano group, a nitro group, a carboxyl group and a sulfo group.

Examples of the cycloalkyl group include a cyclopentyl group and a cyclohexyl group. Examples of the alkenyl group include allyl group, 1-butenyl group and 1-pentenyl group. Examples of the aryl group include a phenyl group and a naphthyl group and the aryl group may have substituents. Examples of the substituent include an alkyl group having 1 to 8 carbon atoms (e.g., methyl group, ethyl group, butyl group) , an alkoxy group having 1 to 6 carbon atoms (e.g., methoxy group, ethoxy group), an aryloxy group (e.g., phenoxy group, p-chlorophenoxy group), a halogen atom (e.g., F, Cl, Br), alkoxycarbonyl group (e.g., methoxycarbonyl group, ethoxycarbonyl group), an amino group, alkyl substituted amino group (e.g., methylamino group), an amide group (e.g., acetoamide group), sulfonamide group (e.g., methane sulfoneamide group) , a cyano group, a nitro group, a carboxyl group and a sulfo group. These aryl groups preferably have 6 to 12 carbon atoms.

A particularly preferable substituent for R₁ and R₂ is an unsubstituted alkyl group, a cyano-substituted alkyl group, an alkoxy-substituted alkyl group and an allyl group. Examples of the unsubstituted alkyl group include (C1-C8) alkyl groups such as a methyl group, an ethyl group, an n-propyl group, an isopropyl group, an n-butyl group, a sec-butyl group, a tert-butyl group, a pentyl group, a hexyl group and a heptyl group. Examplesofthecyano-substitutedalkylgroupinclude cyano-substituted (C1-C6) alkyl groups such as a cyanomethyl group, 2-cyanoethyl group, 3-cyanopropyl group, 2-cyanopropyl group, 4-cyanobutyl group, 3-cyanobutyl group, 2-cyanobutyl group, 5-cyanopentyl group, 4-cyanopentyl group, 3-cyanopentyl group and 2-cyanopentyl group. Examples of the alkoxy-substituted alkyl group include alkoxy-substituted(C1-C6)alkyl groups such as methoxyethyl group, ethoxyethyl group, 3-methoxypropyl group, 3-ethoxypropyl group, 4-methoxybutyl group, 4-ethoxybutyl group, 5-ethoxypentyl group and 5-methoxypentyl group.

In consideration of the heat resistance and moisture heat resistance, either R₁ or R₂ is more preferably a cyano-substituted(C1-C6)alkyl group. Examples of the cyano-substituted (C1-C6) alkyl group are as mentioned above, and more preferably a cyanopropyl group.

m and n indicate the number of substituents of R₁ and R₂ introduced, which are integers of 0-8 satisfying the relationship: m+n=8.

In the formula (3) , X is an atomic group having a negative electric charge(s) necessary for neutralizing a positive electric charge (s). As examples thereof, a monovalent anion such as monovalent organic acid anions and monovalent inorganic acid anions can be mentioned. Examples of the monovalent organic acid anions include organic carboxylate ions such as an acetate ion, a lactate ion, a trifluoroacetate ion, a propionate ion, a benzoate ion, an oxalate ion, a succinate ion and a stearate ion; organic sulfonate ions such as a methanesulfonate ion, a toluenesulfonate ion, a naphthalenemonosulfonate ion, a chlorobenzesulfonate ion, a nitrobenzenesulfonate ion, a dodecylbenzenesulfonate ion, a benzenesulfonate ion, an ethanesulfonate ion and a trifluoromethanesulfonate ion; and organic borate ions such as a tetraphenylborate ion and a butyltriphenylborate, and halogenoalkylsulfonate ions such as a trifluoromethanesulfonate ion and a toluenesulfonate ion or alkylarylsulfonate ions are preferable.

Examples of the monovalent inorganic anion include halogen ions such as a fluorine ion, a chlorine ion, a bromine ion and an iodine ion; a thiocyanate ion, a hexafluoroantimonate ion, a perchlorate ion, a periodate ion, a nitrate ion, a tetrafluoroborate ion, a hexafluorophosphate ion, a molybdate ion, a tungstate ion, a titanate ion, a vanadate ion, a phosphate ion, a borate ion and the like, and a perchlorate ion, an iodine ion, a tetrafluoroborate ion, a hexafluorophosphate ion and a hexafluoroantimonate ion are preferable.

Examples of the divalent anion include naphthalene disulfonic acid derivatives such as naphthalene-1,5-disulfonic acid, R acid, G acid, H acid, benzoyl H acid, p-chlorobenzoyl H acid, p-toluenesulfonyl H acid, chloro H acid, chloroacetyl H acid, metanilic γ acid, 6-sulfonaphthyl-γ acid, C acid, ε acid, p-toluenesulfonyl R acid, naphthalene-1,6-disulfonic acid and 1-naphthol-4,8-disulfonic acid, divalent organic acid ions such as carbonyl J acid, 4,4'-diaminostilbene-2,2'-disulfonic acid, di J acid, naphthalic acid, naphthalene-2,3-dicarboxylic acid, diphenic acid, stilbene-4,4'-dicarboxylic acid, 6-sulfo-2-oxy-3-naphthoic acid, anthraquinone-1,8-disulfonic acid, 1,6-diaminoanthraquinone-2,7-disulfonic acid, 2-(4-sulfophenyl)-6-aminobenzotriazole-5-sulfonic acid, 6-(3-methyl-5-pyrazolonyl)-naphthalene-1,3-disulfonic acid and 1-naphthol-6-(4-amino-3-sulfo)anilino-3-sulfonic acid.

In the aminium salt compound, when the anion is monovalent, one molecule is necessary. When the anion is divalent as in 1,5-naphthalene disulfonic acid, 1/2 molecule is necessary.

Among these atomic groups having a negative electric charge(s), a perchlorate ion, an iodine ion, a tetrafluoroborate ion, a hexafluorophosphate ion, a hexafluoroantimonate ion, trifluoromethanesulfonate ion and toluenesulfonate ion are preferable.

Specific examples of the aminium salt compound of the present invention represented by the formula (3) are shown in Tables 4-6. In Tables 4-6, 1,5-NpS represents 1, 5-naphthalene disulfonic acid and TsO represents a toluene sulfonate ion. Unsubstituted A is simply described as "4H" and the substituted position in A is described as ortho (o-) or meta (m-) with one of the nitrogen atoms of the phenylenediamine skeleton as a center. The substituted position in B is described as 1-4 clockwise relative to the nitrogen atom that bonds to the phenylenediamine skeleton. In addition, regarding (m, n) , a mixture comprising a compound in which m=1, n=7 and a compound in which m=2, n=6 is simply described as (1,7) (2,6).

**TABLE 4**

| No. | R ₁ | R ₂ | A | B | X | (m, n) |
|---|---|---|---|---|---|---|
| 1b | n-C4H9 | C3H6CN | 4H | 4H | SbF6 | (4,4)(3,5)(2,6)(1,7) |
| 2b | n-C4H9 | C3H6CN | 4H | 4H | PF6 | (4,4)(3,5)(2,6)(1,7) |
| 3b | n-C4H9 | C3H6CN | 4H | 4H | PF6 | (6,2)(5,3)(4,4)(3,5) (2,6)(1,7)(0,8) |
| 4b | n-C4H9 | C3H6CN | 4H | 4H | BF4 | (4,4)(3,5)(2,6)(1,7) |
| 5b | n-C4H9 | C3H6CN | 4H | 4H | ClO4 | (2,6)(1,7) |
| 6b | i-C5H11 | C3H6CN | 4H | 4H | SbF6 | (7,1)(6,2)(5,3)(4,4) |
| 7b | i-C5H11 | C3H6CN | 4H | 4H | PF6 | (7,1)(6,2)(5,3)(4,4) |
| 8b | i-C5H11 | C3H6CN | 4H | 4H | BF4 | (7,1)(2,6) |
| 9b | i-C5H11 | C3H6CN | 4H | 4H | ClO4 | (7,1)(6,2) |
| 10b | i-C4H9 | n-C4H9 | 4H | 4H | PF6 | (5,3)(4,4)(3,5) |
| 11b | i-C4H9 | n-C4H9 | 4H | 4H | SbF6 | (7,1)(6,2) |
| 12b | C2H4OCH3 | C3H6CN | 4H | 4H | PF6 | (4,4)(3,5)(2,6)(1,7) |
| 13b | C2H40CH3 | C3H6CN | 4H | 4H | SbF6 | (4,4)(3,5)(2,6)(1,7) |

**TABLE 5**

| No. | R ₁ | R ₂ | A | B | X | (m, n) |
|---|---|---|---|---|---|---|
| 14b | i-C4H9 | C3H6CN | 4H | 4H | SbF6 | (7,1)(6,2)(5,3) |
| 15b | i-C5H11 | i-C4H9 | 4H | 4H | SbF6 | (5,3)(4,4)(3,5) |
| 16b | i-C4H9 | C3H6CN | 4H | 4H | PF6 | (4,4)(3,5)(2,6)(1,7) |
| 17b | CH2CH=CH2 | C3H6CN | 4H | 4H | 1,5-NpS | (7,1)(6,2) |
| 18b | CH2CH=CH2 | C3H6CN | 4H | 4H | PF6 | (4,4)(3,5)(2,6)(1,7) |
| 19b | i-C5H11 | i-C4H9 | 4H | 4H | PF6 | (7,1)(6,2)(5,3)(4,4) |
| 20b | i-C5H11 | n-C4H9 | 4H | 4H | SbF6 | (5,3)(4,4)(3,5) |
| 21b | C2H40CH3 | n-C4H9 | 4H | 4H | PF6 | (4,4)(3,5)(2,6)(1,7) |
| 22b | n-C4H9 | C3H6CN | o-Cl | 4H | PF6 | (7,1)(6,2)(5,3) |
| 23b | n-C4H9 | C3H6CN | m-CH3 | 4H | BF4 | (4,4)(3,5)(2,6)(1,7) |
| 24b | i-C5H11 | C3H6CN | 4H | 2-Cl | ClO4 | (7,1)(6,2) |
| 25b | i-C5H11 | C3H6CN | 4H | 2-CH3 | SbF6 | (7,1)(6,2)(5,3) |

**TABLE 6**

| No. | R ₁ | R ₂ | A | B | X | (m, n) |
|---|---|---|---|---|---|---|
| 26b | Cy-C6H12 | C3H6CN | 4H | 4H | SbF6 | (7,1)(6,2)(5,3) |
| 27b | CH2Ph | n-C4H9 | 4H | 4H | TsO | (4,4)(3,5)(2,6)(1,7) |
| 28b | Ph | n-C4H9 | o-Cl | 4H | ClO4 | (2,6)(1,7) |
| 29b | i-C5H11 | C4H8CN | 4H | 4H | SbF6 | (5,3)(4,4)(3,5) |
| 30b | s-C5H11 | C2H40CH3 | 4H | 4H | PF6 | (2,6)(1,7) |
| 31b | n-C5H11 | C3H6CN | 4H | 4H | PF6 | (8,0)(7,1)(6,2)(5,3) |
| 32b | n-C5H11 | C3H6CN | 4H | 4H | SbF6 | (5,3)(4,4)(3,5) |
| 33b | n-C2H5 | i-C5H11 | 4H | 4H | PF6 | (3,5)(2,6)(1,7)(0,8) |
| 34b | C2H4OPh | n-C4H9 | 4H | 4H | PF6 | (7,1)(6,2) |
| 35b | CH2CH=CH2 | i-C5H11 | 4H | 4H | BF4 | (4,4)(3,5)(2,6)(1,7) |
| 36b | i-C5H11 | C2H40CH3 | 4H | 4H | ClO4 | (4,4)(3,5)(2,6)(1,7) |
| 37b | i-C5H11 | C3H6CN | o-Cl | 4H | SbF6 | (7,1)(6,2)(5,3) |

The mixture of aminium salt compounds represented by the formula (3) of the present invention can be obtained by the following method described, for example, in Japanese Patent Publication (KOKOKU) No. 43-25335. That is, an amino compound represented by the following formula (4) 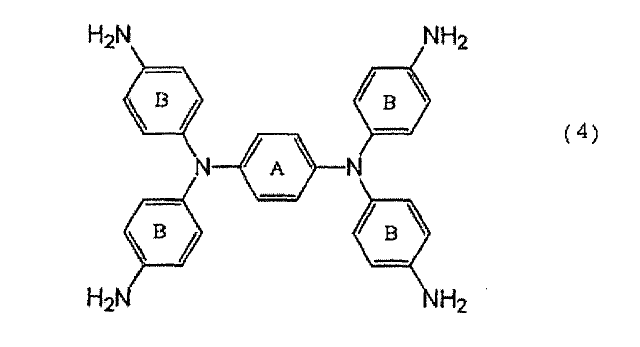 wherein ring A and ring B are as defined above, obtained by a Ullmann reaction and reduction reaction, is mixed, in an organic solvent, preferably an aqueous polar solvent such as DMF, DMI or NMP, at 30 to 160°C, preferably 50 to 140°C, with a halogenated compound corresponding to R₁ (e.g., BrC4H9 when R₁ is n-C4H9) and a halogenated compound corresponding to R₂ (e.g., BrCH2CH(CH3)2 when R₂ is i-C4H9) in any proportion, and reacted to obtain a mixture of compounds represented by the formula (1). To control the ratio of m to n, it is also possible to synthesize a mixture of compounds in any proportion by reacting the halogenated compound corresponding to R₁ in advance and then reacting the halogenated compound corresponding to R₂.

Then the mixture of compounds synthesized above is subjected to oxidization reaction by adding 1 equivalent of anoxidant (e.g., silversalt) correspondingtoXoftheformula (3) at 0 to 100°C, preferably 5 to 70°C, using an organic solvent, preferably an aqueous polar solvent such as DMF, DMI or NMP, to obtain a mixture of aminium salt compounds represented by the formula (3) of the present invention.

### Near-infrared absorption filter

The near-infrared absorption filter of the present invention may be one having, on a substrate, a layer containing the aforementioned mixture of diimonium salt compounds, or the substrate itself may be a layer comprising a resin composition (or a cured product thereof) containing the aforementioned mixture of diimonium salt compounds. As the substrate, a substrate made of resin is usually used without particular limitation. The thickness of the diimonium salt compound containing layer is generally about 0.1 µm to 10 mm and suitably determined according to purposes such as near-infrared cut-off ratio. The content of the mixture is also suitably determined depending on the target near-infrared cut-off ratio.

As the resin for the substrate, a resin having a transparency as high as possible when formed into a resin sheet or a resin film is preferable. Specific examples thereof include vinyl compounds such as polyethytlene, polystyrene, polyacrylic acid, polyacrylic acid ester, polyvinyl acetate, polyacrylonitrile, polyvinyl chloride and polyvinyl fluoride, and addition polymers of these vinyl compounds, a copolymer of vinyl compounds or fluorine compounds of polymethacrylic acid, polymethacrylate, polyvinylidene chloride, ployvinylidene fluoride, polyvinylidence cyanide, vinylidene fluoride/trifluoroethylene copolymer, vinylidene fluoride/tetrafluoroethylene copolymer and vinylidene cyanide/vinyl acetate copolymer, fluorine containing resins such as polytrifluoroethylene, polytetrafluoroethylene and polyhexafluoropropylene, polyamides such as Nylon 6 and Nylon 66, polyimide, polyurethane, polypeptide, polyesters such as polyethylene terephthalate, polycarbonate, polyethers such as polyoxymethylene, epoxy resins, polyvinyl alcohols and polyvinyl butyral.

The method for producing the near-infrared absorption filter of the present invention is not particularly limited and for example, the following methods can be employed. For example, (1) a method of producing a resin sheet or film by kneading the aforementioned mixture of diimonium salt compounds into a resin(s) and then thermally molding, (2) a method of producing a resin sheet or film by subjecting the aforementioned mixture and resin monomers or a prepolymer of the resin monomers to cast polymerization in the presence of a polymerization catalyst, (3) a method comprising preparing coating which contains the mixture and applying it on a transparent resin sheet, a transparent film or a transparent glass sheet, and (4) a method comprising incorporating the mixture into an adhesive agent to produce a ply resin sheet, a ply resin film or a ply glass sheet.

As the method (1), while the processing temperature and film forming conditions (or resin sheet forming conditions) are somewhat different depending on the resin to be used, amethod comprising adding themixture of the present invention to powder or pellets of a substrate resin, heating and melting the resulting mixture at a temperature of 150 to 350°C and then molding it into a resin sheet, or a method of film forming (resin sheet forming) using an extruder can be mentioned. The amount of the aforementioned mixture of diimonium salt compounds to be added varies depending on the thickness of resin sheet or film to be prepared, absorption intensity and visible light transmittance, but the amount is generally 0.01 to 30 wt %, preferably 0.03 to 15 wt % relative to the weight of the binder resin.

In the method (2) of producing a resin sheet or film by subjecting the aforementioned mixture and resin monomers or a prepolymer of the resin monomers to cast polymerization in the presence of a polymerization catalyst, the mixture containing these is put into a mold to cure through a reaction, or the mixture is molded by casting into a metal mold and leaving it until it is hardened. Many kinds of resins can be molded by this step and specific examples thereof include an acrylic resin, diethyleneglycolbis (allylcarbonate) resin, epoxy resin, phenol-formaldehyde resin, polystyrene resin and silicon resin. Of these methods, a casting method employing a bulk polymerization of methyl methacrylate through which an acrylic sheet excellent in hardness, heat resistance and chemical resistance is obtained is preferable.

As the polymerization catalyst, known radical thermal polymerization initiators can be used. Examples thereof include peroxides such as benzoyl peroxide, p-chlorobenzoyl peroxide and diisopropylperoxycarbonate and azo compounds such as azobisisobutylonitrile. The amount to be used is generally 0.01 wt % to 5 wt % relative to the total amount of the mixture. In the thermal polymerization, the heating temperature is generally 40 to 200°C and the polymerization time is generally about 30 minutes to 8 hours. In addition to the thermal polymerization, a photopolymerization method using a photo-initiator or a sensitizer can also be employed.

The method (3) includes a method in which the mixture to be used in the present invention is dissolved in a binder resin (s) and an organic solvent to form coating, and a method in which the mixture of the present invention is pulverized into fine particles and dispersed to form an aqueous coating. In the former method, an aliphatic ester resin, an acrylic resin, a melamine resin, an urethane resin, an aromatic ester resin, a polycarbonate resin, a polyvinyl resin, an aliphatic polyolefin resin, an aromatic polyolefin resin, a polyvinyl alcohol resin and a polyvinyl modified resin or a copolymer resin thereof can be used as a binder.

As the solvent, a halogen solvent, an alcohol solvent, a ketone solvent, an ester solvent, an aliphatic hydrocarbon solvent, an aromatic hydrocarbon solvent, an ether solvent or a mixed solvent thereof can be used. The concentration of the near-infrared absorption compound of the present invention varies depending on the thickness, absorption intensity and visible light transmittance of the coating to be prepared, but the concentration is generally 0.1 to 30 wt % relative to the binder resin.

A near-infrared absorption filter can be obtained by applying the coating prepared as above on a transparent resin film, a transparent resin sheet or transparent glass using a spin coater, a bar coater, a roll coater or a spray.

As an adhesive in the method (4), adhesives for resin such as a general silicon adhesive, urethane adhesive and acrylic adhesive, adhesives for ply glass such as polyvinyl butyral adhesive, known transparent adhesives for ply glass such as ethylene-vinyl acetate adhesive can be used. A filter is prepared by adhering transparent resin sheets, a resin sheet and a resin film, a resin sheet and a glass sheet, resin films, a resin film and a glass sheet, or glass sheets by using an adhesive to which 0.1 to 30 wt% of the compound of the present invention is added.

In each method, additives generally used in the molding of a resin, such as an ultraviolet ray absorber and a plasticizer may be added when kneading and mixing.

Those obtained by adding a mixture of the compounds represented by the formula (2) to a resin according to the methods (1) to (4) described above are the near-infrared absorption composition of the present invention.

The near-infrared absorption filter of the present invention may contain the mixture of the diimonium salt compounds represented by the formula (2) alone as a near-infrared absorption compound, but the filter may be produced by combining with another near-infrared absorption compound. Examples of other infrared absorbing compounds include a phthalocyanine dye, a cyanine dye and a dithiol nickel complex. In addition, as an inorganic metal infrared absorbing compound, metal copper, copper compounds such as copper sulfide and copper oxide, metal mixtures containing zinc oxide as a main component, a tungsten compound, ITO and ATO can be mentioned.

In order to change the color tone of the filter, a dye having absorption in the visible region is preferably added within a range that does not impair the effect of the present invention. Alternatively, a filter containing a dye for adjusting color tone alone can be prepared and adhered afterward.

When such filter is used as a front sheet of a display, the higher the visible light transmittance, the better, and the visible light transmittance needs to be not less than 40%, preferably not less than 50%. A cut-off region of near-infrared is usually 800 to 1000 nm, more preferably 800 to 900 nm, and the average transmittance in this region is not more than 50%, more preferably not more than 30%, further preferably not more than 20% and particularly preferably not more than 10%.

The near-infrared absorption filter of the present invention can be used for not only front sheets of displays but also filters and films that should cut infrared rays, such as heat insulating films and sunglasses.

The near-infrared absorption filter of the present invention is a superior filter capable of absorbing in a wide range in the near-infrared region, while showing a extremely high transmittance in the visible light region. The near-infrared absorption filter of the present invention has a sufficient solubility and superior processability compared to a conventional near-infrared absorption filter comprising a diimonium salt. It is particularly superior in heat resistance, moisture heat resistance and light resistance, and a near-infrared absorption filter which is difficult to cause a reaction such as decomposition during a heat resistance test and which hardly suffer from coloring of the visible section can be obtained. Due to these characteristics, the composition of the present invention can be suitably used for a near-infrared absorption filter, a near-infrared absorption film such as a heat insulating film and sunglasses, and is particularly suitable for a near-infrared absorption filter for a plasma display.

The near-infrared absorption filter of the present invention may contain, instead of or in addition to the aforementioned mixture of diimonium salt compounds, a mixture of aminium salt compounds represented by the formula (3). The method of producing a near-infrared absorption filter which contains the mixture of aminium salt compounds, other additives and the like are the same as those containing diimonium salt compounds described above.

### Optical information recording medium

The optical information recording medium of the present invention comprises a recording layer on a substrate and the recording layer is characterized in that it contains the aforementioned mixture of diimonium salt compounds of the formula (2) or the aforementioned mixture of aminium salt compounds of the formula (3). The recording layer may contain a mixture of diimonium salt compounds alone or a mixture of aminium salt compounds alone, or both mixtures in combination. The recording layer may be composed of a mixture of diimonium salt compounds and/or a mixture of aminium salt compounds alone, or a mixture with various additives such as a binder. In this case, information is recorded by means of the mixture of diimonium salt compounds of the formula (2) and/or the mixture of aminium salt compounds the formula (3).

By incorporating the mixture of diimonium salt compounds and/or the mixture of aminium salt compounds into the recording layer of an optical information recording medium, on which information is recorded by an organic dye, the light resistance of the optical information recording medium can be improved. Such optical information recording medium is one kind of the optical information recording media of the present invention.

In the optical information recording medium, examples of the organic dye that can be used together with the mixture of diimonium salt compounds and/or the mixture of aminium salt compounds of the present invention include generally known dyes such as a cyanine dye, a squalilium dye, an indoaniline dye, a phthalocyanine dye, an azo dye, a merocyanine dye, a polymethine dye, a naphthoquinone dye and a pyririum dye.

The mixture of diimonium salt compounds and/or the mixture of aminium salt compounds is generally used in an amount of 0.01 to 10 mol, preferably in an amount of 0.03 to 3 mol relative to 1 mol of the organic dye.

According to the optical information recording medium of the present invention, a recording layer comprising a mixture of diimonium salt compounds of the formula (2) and/or a mixture of aminium salt compounds of the formula (3), and optionally a dye is provided on a substrate, and where necessary, a reflective layer or a protective layer is formed thereon. As the substrate, any known substrate can be used. Examples thereof include a glass sheet, a metal sheet, a plastic sheet or film. Examples of plastics for preparing such substrate include acrylic resin, polycarbonate resin, methacrylic resin, polysulfone resin, polyimide resin, non-crystalline polyolefin resin, polyester resin and polypropylene resin. As to the shape of the substrate, various kinds of shapes including a disk, a card, a sheet and a roll film can be employed.

Guide grooves may also be formed on a glass or plastic substrate to facilitate tracking in recording. An undercoating layer made of a plastic binder, an inorganic oxide or an inorganic sulfide may be formed on the glass or plastic substrate, and an undercoating layer having a thermal conductivity lower than that of the substrate is preferable.

The recording layer of the optical information recording medium of the present invention can be obtained by, for example, dissolving a mixture of diimonium salt compounds of the formula (2) and/or a mixture of aminium salt compounds of the formula (3), more preferably a mixture of diimonium salt compounds of the formula (2) and/or a mixture of aminium salt compounds of the formula (3) and another organic dye, in a known organic solvent such as tetrafluoropropanol (TFP), octafluoropentanol (OFP), diacetone alcohol, methanol, ethanol, butanol, methylcellosolve, ethylcellosolve, dichloroethane, isophorone and cyclohexanone, and if necessary adding a suitable binder, and applying the resulting solution on the substrate by a spin coater, a bar coater or a roll coater. As other methods, the recording layer can be obtained by a vacuum evaporation method, a sputtering method, a doctor blade method, a casting method or a dipping method in which the substrate is dipped into a solution. As the binder, acrylic resin, urethane resin and epoxy resin can be used.

The thickness of the recording layer is preferably 0.01 µm to 5 µm, more preferably 0.02 µm to 3 µm, in view of the recording sensitivity and reflectance.

In the optical information recording medium of the present invention, an undercoating layer may be formed under the recording layer, and a protective layer may be formed on the recording layer, if necessary. Furthermore, a reflective layer may be formed between the recording layer and the protective layer. When a reflective layer is formed, the reflective layer is made of a metal such as gold, silver, copper or aluminum, preferably gold, silver or aluminum. These metals may be used either alone or as an alloy of two or more kinds. The reflective layer is formed by a vacuum evaporation method, a sputtering method or an ion plating method. The thickness of the reflective layer is 0.02 to 2µm. The protective layer that may be formed on the reflective layer is generally formed by applying an ultraviolet curable resin by a spin coating method and then irradiating with ultraviolet ray to cure the coated film. In addition, an epoxy resin, an acrylic resin, a silicone resin and urethane resin can be used as a material for forming a protective film. The thickness of the protective layer is usually 0.01 to 100 µm.

Recording of information and formation of an image on the optical recording medium of the present invention are conducted by irradiating a recording layer with a collected, spot-like high energy beam of laser rays such as semiconductor laser, helium-neon laser, He-Cd laser, YAG laser and Ar laser through the substrate or from the other side of the substrate, while reading of information or an image is conducted by irradiating with a low output laser beam to detect the difference in the amounts of reflected light or transmitted light between the pit section and the section wherein no pit is formed.

The optical information recording medium of the present invention is a superior optical information recording medium in which high lightfastness is achieved by incorporating a mixture of compounds of the formula (2) and/or a mixture of compounds of the formula (3). These compounds have sufficient solubility and superior processability. In particular, in the optical information recording medium containing an organic dye in the recording layer, can be used as a light stabilizer. By using these compounds as a light stabilizer, an optical information recording medium having an extremely superior lightfastness can be obtained.

The mixture of diimonium salt compounds of the present invention has a maximum absorption wavelength of not less than 900 nm and a large absorption peak with an absorption coefficient of several ten to hundred thousand. The results of the stability test for heat resistance and moisture heat resistance indicate that the mixture of diimonium salt compounds of the present invention shows a smaller color change and a higher stability, as compared with the conventional compositions. The results of a solubility test reveal that the mixture can be used as an infrared absorbing agent having a sufficient solubility for solvents.

The mixture of aminium salt compounds of the present invention has a maximum absorption wavelength of not less than 850 nm and a large absorption peak with an absorption coefficient of several ten thousand. The results of a solubility test reveal that the mixture can be used as an infrared absorbing agent having a higher solubility and superior processability as compared with the conventional composition. The results of light resistance test indicate that the light resistance can be greatly improved by adding the mixture of the compounds of the present invention to an organic dye.

The near-infrared absorption filter of the present invention has a sufficient solubility, superior processability, and in addition, superior stability such as heat resistance, moisture heat resistance and light resistance as compared with the conventional near-infrared absorption filter comprising a diimonium salt. In particular, it is a near-infrared absorption filter superior in heat resistance, moisture heat resistance and light resistance, which is difficult to cause a reaction such as decomposition during these stability tests and which hardly suffer from coloring of the visible section. Due to these characteristics, the composition of the present invention can be suitably used for a near-infrared absorption filter, a near-infrared absorption film such as a heat insulating film and sunglasses, and is particularly suitable for a near-infrared absorption filter for a plasma display.

According to the optical information recording medium of the present invention, by incorporating a mixture of compounds of the formula (2) and/or a mixture of compounds of the formula (3), the light resistance can be greatly improved as compared with the conventional information recording medium containing a diimonium salt. And these compounds have sufficient solubility and superior processability. When this compound is incorporated, for example, into a thin film of an organic dye which corresponds to the recording layer of an optical information recording medium, an optical information recording medium having greatly improved durability in repeat playback and lightfastness can be provided.

### Examples

In the following, the present invention is explained in detail by means of Examples but the present invention is not limited to these Examples. In Examples, "part (s) " means "part(s) by weight" unless otherwise specified.

### Preparation of diimonium salt compound

### Example 1

### Substitution reaction

To 16 parts of DMF were added 5.3 parts of N,N,N',N'-tetrakis(aminophenyl)-p-phenylenediamine, 20 parts of potassium carbonate, 10 parts of potassium iodide, 12 parts of 1-chlorobutane which is a regent introduced as R₁ and 14 parts of 4-chlorobutylonitorile which is a regent introduced as R₂. Reaction was conducted at 90°C for 3 hours and then at 130°C for an hour. After cooling, the resulting mixture was filtrated and 40 parts of methanol was added to the reaction mixture, followed by stirring at not more than 5°C for an hour. Generated crystals were filtrated, washed with methanol and dried to obtain 5.1 parts of light brown crystals. Assuming that R₁ was n-C4H9 group and R₂ was C3H6CN group from the measurement of the molecular weight, the mixture had (m,n)=(4,4) (3,5) (2,6) (1,7).
melting point (DSC) 150°C

### Oxidation reaction

0.92 part of the mixture synthesized above was added to 5 parts of DMF and after heating at 60°C to dissolve the mixture, 0.72 part of silver hexafluoroantimonate dissolved in 5 parts of DMF was added and reaction was conducted for 30minutes. After cooling, precipitated silver was filtered. 20 parts of water was added dropwise to the reaction mixture slowly and after the addition, stirring was conducted for 15 minutes. Generated black crystals were filtrated and washed with 50 parts of water, and the obtained cake was dried to obtain 1.3 parts of mixture of compounds No.1a.
No.1a: λmax 1086 nm (dichloromethane)
absorption coefficient 110,000

Synthesis was conducted in the same manner except that silver hexafluorophosphate was used instead of silver hexafluoroantimonate used in the above oxidation reaction to obtain 1.1 parts of mixture of compounds No.2a.
No.2a: λmax 1086 nm (dichloromethane)
absorption coefficient 108,000

### Example 2

Synthesis was conducted in the same manner except that 6.2 parts of 1-bromobutane was used as R₁ and 23.2 parts of chlorobutylonitrile was used as R₂ instead of 1-chlorobutane and 4-chlorobutylonitrile, as reagents for substitution in the substitution reaction of the above Example 1, and light brown crystals were obtained. The obtained mixture of compounds was subjected to the same measurement using LC (HP-1100, made by Agilent Ltd.) and a mass spectrometer (LCT, made by Micromass Ltd.) and when assuming that R₁ was n-C4H9 group and R₂ was C3H6CN group, the mixture had (m,n)=(5,3) (4,4) (3,5) (2,6) (1,7) and the ratio was 8%, 36%, 35%, 17% and 4% from the left.
melting point (DSC) 144°C

Synthesis was conducted by using this compound, reacting with 2 equimolar amounts of silver hexafluorophosphate as in the oxidation reaction of Example 1 to obtain compound No.3a.
No.3a: λmax 1090 nm
absorption coefficient 103,000 (dichloromethane)

### Example 3

Synthesis was conducted in the same manner except that 1-bromo-3-methylbutane was used as R₁ and 4-chlorobutylonitrile was used as R₂ instead of 1-chlorobutane and 4-chlorobutylonitrile, as reagents for substitution in the substitution reaction of the above Example 1, and light brown crystals were obtained. The obtained mixture of compounds was subjected to the same measurement as above and when assuming that R₁ was i-C5H11 group and R₂ was C3H6CN group, the mixture had (m,n)=(7,1) (6,2) (5,3) (4,4).
melting point (DSC) 95°C

Synthesis was conducted by using this mixture, reacting with silver hexafluoroantimonate or silver hexafluorophosphate as in the oxidation reaction of Example 1 to obtain mixtures of compound No.6a and compounds No.7a, respectively.
No.6a: λmax 1104 nm (dichloromethane)
absorption coefficient 106,000
No.7a: λmax 1104 nm (dichloromethane)
absorption coefficient 106,000

### Example 4

Synthesis was conducted in the same manner except that 1-bromo-2-methylpropane was used as R₁ and 4-chlorobutylonitrile was used as R₂ instead of 1-chlorobutane and 4-chlorobutylonitrile, as reagents for substitution in the substitution reaction of the above Example 1, and light brown crystals were obtained. When assuming that R₁ was i-C4H9 group and R₂ was C3H6CN group, the obtained compound was a mixture which had (m,n)=(4,4) (3, 5) (2, 6) (1,7).
melting point (DSC) 146°C

Synthesis was conducted by using this mixture, reacting with silver hexafluoroantimonate or silver hexafluorophosphate as in the oxidation reaction of Example 1 to obtain mixture of compounds No.14a and mixture of compounds No.16a, respectively.
No.14a: λmax 1080 nm (dichloromethane)
absorption coefficient 109,000
No.16a: λmax 1080 nm (dichloromethane)
absorption coefficient 106,000

### Example 5

Synthesis was conducted in the same manner except that 1-bromo-3-methylbutane was used as R₁ and 1-bromo-2-methylpropane was used as R₂ instead of 1-chlorobutane and 4-chlorobutylonitrile, as reagents for substitution in the substitution reaction of the above Example 1, and light brown crystals were obtained. When assuming that R₁ was i-C5H11 group and R₂ was i-C4H9 group, the obtained compound was a mixture which had (m,n)=(7,1) (6,2) (5,3) (4,4).
melting point (DSC) 90°C

Synthesis was conducted by using this mixture, reacting with silver hexafluoroantimonate or silver hexafluorophosphate as in the oxidation reaction of Example 1 to obtain mixtures of compound No.15a and compounds No. 19a, respectively.
No.15a: λmax 1106 nm (dichloromethane)
absorption coefficient 103,000
No.19a: λmax 1104 nm (dichloromethane)
absorption coefficient 109,000

### Example 6

Synthesis was conducted in the same manner except that 1-bromo-2-methoxyethane was used as R₁ and 4-chlorobutylonitrile was used as R₂ instead of 1-chlorobutane and 4-chlorobutylonitrile, as reagents for substitution in the substitution reaction of the above Example 1, and light brown crystals were obtained. When assuming that R₁ was C2H4OCH3 group and R₂ was C3H6CN group, the obtained compound was a mixture which had (m,n)=(4,4) (3,5) (2,6) (1,7).
melting point (DSC) 138°C

Synthesis was conducted by using this mixture, reacting with silver hexafluoroantimonate or silver hexafluorophosphate as in the oxidation reaction of Example 1 to obtain mixture of compounds No. 13a andmixture of compounds No.12a, respectively.
No.13a: λmax 1082 nm (dichloromethane)
absorption coefficient 108,000
No.12a: λmax 1082 nm (dichloromethane)
absorption coefficient 107,000

Other compounds can be synthesized in the same manner as in the above Example 1, by synthesizing corresponding phenylenediamine derivatives, oxidizing the same with corresponding various oxidants as mentioned above such as silver salts and reacting with corresponding anions.

### Preparation of aminium salt compound

### Example 7

### Substitution reaction

To 16 parts of DMF were added 5.3 parts of N,N,N',N'-tetrakis(aminophenyl)-p-phenylenediamine, 20 parts of potassium carbonate, 10 parts of potassium iodide, 12 parts of 1-chlorobutane which is a regent introduced as R₁ and 14 parts of 4-chlorobutylonitorile which is a regent introduced as R₂. Reaction was conducted at 90°C for 3 hours and then at 130°C for an hour. After cooling, the resulting mixture was filtrated and 40 parts of methanol was added to the reaction mixture, followed by stirring at not more than 5°C for an hour. Generated crystals were filtrated, washed with methanol and dried to obtain 5.1 parts of light brown crystals. From the measurement of the molecular weight, when assuming that R₁ was n-C4H9 group and R₂ was C3H6CN group, the obtained mixture had (m,n)=(4,4) (3,5) (2,6) (1,7).
melting point (DSC) 150°C

### Oxidation reaction

0.92 part of the mixture synthesized above was added to 5 parts of DMF and after heating at 60°C to dissolve the mixture, 0.36 part of silver hexafluoroantimonate dissolved in 5 parts of DMF was added and reaction was conducted for 30 minutes. After cooling, precipitated silver was filtered. 20 parts of water was added dropwise to the reaction mixture slowly and after the addition, stirring was conducted for 15 minutes. Generated black crystals were filtrated and washed with 50 parts of water, and the obtained cake was dried to obtain 1.1 parts of mixture of compounds No.1b.
No.1b: maximum absorption wavelength 908 nm (acetone)
absorption coefficient 20,000

Synthesis was conducted in the same manner except that silver hexafluorophosphate was used instead of silver hexafluoroantimonate used in the above oxidation reaction. to obtain 1.1 parts of mixture of compounds No.2b.
No.2b: maximum absorption wavelength 918 nm (acetone)
absorption coefficient 21,500

Synthesis was conducted in the same manner except that silver tetrafluoroborate was used instead of silver hexafluoroantimonate used in the above oxidation reaction to obtain 1.0 part of mixture of compounds No.4b.
No.4b: maximum absorption wavelength 918 nm (acetone)
absorption coefficient 19,300

### Example 8

Synthesis was conducted in the same manner except that 4.9 parts of 1-bromobutane was used as R₁ and 24.2 parts of chlorobutylonitrile was used as R₂, as reagents for substitution in the substitution reaction of the above Example 7, and light brown crystals were obtained. The obtained mixture of compounds was subjected to the same measurement using LC (HP-1100, made by Agilent Ltd.) andamass spectrometer (LCT, made by Micromass Ltd.) and when assuming that R₁ was n-C4H9 group and R₂ was C3H6CN group, the mixture had (m,n)=(6,2) (5,3) (4,4) (3,5) (2,6) (1,7) (0,8) and the ratio was 3%, 11%, 21%, 27%, 23%, 11% and 4% from the left.
melting point (DSC) 149°C

Synthesis was conducted by using this compound, reacting with 1 equimolar amount of silver hexafluorophosphate as in the oxidation reaction of Example 7 to obtain compound No. 3b. No.3a: λmax 918 nm
absorption coefficient 21,000 (acetone)

### Example 9

Synthesis was conducted in the same manner except that 1-bromo-3-methylbutane was used as R₁ and 4-chlorobutylonitrile was used as R₂ instead of 1-chlorobutane and 4-chlorobutylonitrile, as reagents for substitution in the substitution reaction of the above Example 7, and light brown crystals were obtained. The obtained mixture of compounds was subjected to the same measurement as above and when assuming that R₁ was i-C5H11 group and R₂ was C3H6CN group, the mixture had (m,n)=(7,1) (6,2) (5,3) (4,4).
melting point (DSC) 95°C

Synthesis was conducted by using this mixture, reacting with silver hexafluoroantimonate or silver hexafluorophosphate as in the oxidation reaction of Example 7 to obtain mixture of compounds No.6b and mixture of compounds No.7b, respectively.
No.6b: maximum absorption wavelength 950 nm (acetone)
absorption coefficient 23,300
No.7b: maximum absorption wavelength 956 nm (acetone)
absorption coefficient 24,000

### Example 10

Synthesis was conducted in the same manner except that 1-bromo-2-methylpropane was used as R₁ and 4-chlorobutylonitrile was used as R₂ instead of 1-chlorobutane and 4-chlorobutylonitrile, as reagents for substitution in the substitution reaction of the above Example 7, and light brown crystals were obtained. When assuming that R₁ was i-C4H9 group and R₂ was C3H6CN group, the obtained compound was a mixture which had (m,n)=(4,4)(3,5)(2,6)(1,7).
melting point (DSC) 146°C

Synthesis was conducted by using this mixture, reacting with silver hexafluoroantimonate as in the oxidation reaction of Example 7 to obtain mixture of compounds No.14b. No.14b: maximum absorption wavelength 908 nm (acetone)
absorption coefficient 19,000

### Example 11

Synthesis was conducted in the same manner except that 1-bromo-3-methylbutane was used as R₁ and 1-bromo-2-methylpropane was used as R₂ instead of 1-chlorobutane and 4-chlorobutylonitrile, as reagents for substitution in the substitution reaction of the above Example 7, and light brown crystals were obtained. When assuming that R₁ was i-C5H11 group and R₂ was i-C4H9 group, the obtained compound was a mixture which had (m,n)=(7,1) (6,2) (5,3) (4,4). melting point (DSC) 90°C

Synthesis was conducted by using this mixture, reacting with silver hexafluoroantimonate as in the oxidation reaction of Example 7 to obtain mixture of compounds No.15b. No.15b: maximum absorption wavelength 958 nm (acetone)
absorption coefficient 24,000

### Example 12

Synthesis was conducted in the same manner except that 1-bromo-2-methoxyethane was used as R₁ and 4-chlorobutylonitrile was used as R₂ instead of 1-chlorobutane and 4-chlorobutylonitrile, as reagents for substitution in the substitution reaction of the above Example 7, and light brown crystals were obtained. When assuming that R₁ was C2H4OCH3 group and R₂ was C3H6CN group, the obtained compound was a mixture which had (m,n)=(4,4) (3,5) (2,6) (1,7).
melting point (DSC) 138°C

Synthesis was conducted by using this compound, reacting with silver hexafluoroantimonate or silver hexafluorophosphate as in the oxidation reaction of Example 7 to obtain compound No.13b and compound No. 12b, respectively.
No.13b: maximum absorption wavelength 902 nm (acetone)
absorption coefficient 19,600
No.12b: maximum absorption wavelength 910 nm (acetone)
absorption coefficient 21,700

Other compounds can be synthesized in the same manner as in the above Example 7, by synthesizing corresponding phenylenediamine derivatives, oxidizing the same with corresponding various oxidants as mentioned above such as silver salts and reacting with corresponding anions.

### Experimental Example 1

### Solubility test of diimonium salt compound

Solubility of compounds No.1a, No.3a, No.6a, No.2a, No.7a, No. 13a and No. 12a obtained in the above Examples in diacetone alcohol (DAA), methyl ethyl ketone (MEK) and cyclohexanone was measured. Measurement of solubility was conducted in the same manner except that hexafluoroantimonate of tetrakis{p-di(3-cyanopropyl)aminophenyl} phenylenediimonium (Comparative Sample 1) was used instead of the above compounds. The results of the solubility tests are shown in Table 7.

**Table 7**

| (solubility test) | | | |
|---|---|---|---|
| solubility (wt%) | | | |
| | DAA | MEK | cyclohexane |
| No.1a | 0.6 | 2.2 | 0.5 |
| No.3a | 0.6 | 1.8 | 0.6 |
| No.6a | 5.0 | 4.4 | 3.0 |
| No.2a | 0.6 | 1.4 | 0.5 |
| No.7a | 9.3 | 1.6 | 3.4 |
| No.13a | 4.0 | not less than 10 | not less than 10 |
| No.12a | 2.2 | not less than 10 | 9.2 |
| Comparative Sample 1 | insoluble | insoluble | insoluble |

### Experimental Example 2

### Solubility test of aminium salt compound

Solubility of compounds No.1b, No.3b, No.4b, No.6b, No.7b, No.12b, No.13b, No.14b and No.15b obtained in the above Examples in diacetone alcohol (DAA), methyl ethyl ketone (MEK) , tetrahydrofuran (THF) and methylcellosolve was measured. Measurement of solubility was conducted in the same manner except that hexafluoroantimonate of tetrakis{p-di(3-cyanopropyl)aminophenyl}phenyleneaminium (Comparative Sample 2) and hexafluorophosphate thereof (Comparative Sample 3) were used instead of the above compounds. The results of the solubility tests are shown in Table 8.

**Table 8 (solubility test)**

| solubility (wt%) | | | |
|---|---|---|---|
| | DAA | MEK | THF |
| No.1b | not less than 10 | not less than 10 | not less than 10 |
| No.3b | not less than 10 | 9.7 | 10.0 |
| No.4b | 9.7 | not less than 10 | not less than 10 |
| No.6b | 8.0 | 7.2 | 7.2 |
| No.7b | not less than 10 | not less than 10 | not less than 10 |
| No.12b | 8.7 | not less than 10 | not less than 10 |
| No.13b | 8.3 | 6.8 | 6.8 |
| No.14b | 3.2 | not less than 10 | not less than 10 |
| No.15 | 9.9 | 9.8 | 9.8 |
| Comparative Sample 2 | 1.2 | 1.7 | insoluble |
| Comparative Sample 3 | 0.7 | 1.2 | insoluble |

### Experimental Example 3

### Heat resistance, moisture heat resistance and lightfastness test of near-infrared absorption filter containing mixture of diimonium salt compounds

1.2 parts of compound No.1a (Sample 1X), No.6a (Sample 2X) and No.15a (Sample 3X) was each dissolved in 18.8 parts of MEK. To the solution was added 80 parts of a resin solution obtained by dissolving 25 parts of an acrylic resin (Dianal BR-80, available from Mitsubishi Rayon Co., Ltd.) in 75 parts of MEK to prepare a coating solution. The coating solution was applied to a polyester film in a thickness of 2 to 4 µm and dried at 80°C to obtain the near-infrared absorption filter of the present invention.

The obtained coat film was subjected to a heat resistance stability test in a hot air dryer at 80°C for a predetermined time and a moisture heat resistance stability test in a temperature and humidity controlled apparatus under conditions of 60°C and 95%RH for a predetermined time. In addition, Samples 1X and 2X were subjected to a lightfastness test using a weatherometer (Ci4000, made by Atlas Electric Devices Co.) under conditions of light source output: 0.36 W/m², bath temperature: 24°C, black panel temperature: 40°C and humidity: 30%RH for 50 hours. After the tests, the color of the film was measured by an ultraviolet/visible light spectrophotometer (UV-2100, made by Shimadzu Corporation) to calculate L*, a*, b* values and stability was evaluated from the variation of the b* value. The results of heat resistance test are shown in Table 9, the results of the moisture heat resistance test are shown in Table 10 and the results of the lightfastness test are shown in Table 11.

Coat films were prepared and evaluated in the same manner except that hexafluoroantimonate of tetrakis{p-di(n-butyl)aminophenyl}phenylenediimonium (Comparative Sample 1X) and hexafluoroantimonate of tetrakis{p-di(3-cyanopropyl)aminophenyl}phenylenediimoni um (Comparative Sample 2X) were used instead of the above compounds. The results are shown in Tables 9-11.

**Table 9**

| (Heat resistance stability test) b* value | | | | |
|---|---|---|---|---|
| | initial stage | after 4 days | after 7 days | after 14 days |
| Sample 1X | 2.79 | 3.42 | 3.40 | 3.47 |
| Sample 2X | 3.36 | 3.27 | 3.30 | 3.55 |
| Sample 3X | 2.83 | 3.24 | 3.36 | 3.54 |
| Comparative Sample 1X | 2.85 | 3.65 | 3.93 | 4.30 |

Comparative Sample 2X had poor solubility in MEK and it was impossible to prepare a film.

**Table 10**

| (Heat moisture resistance stability test) b* value | | | | |
|---|---|---|---|---|
| | initial stage | after 4 days | after 7 days | after 14 days |
| Sample 1X | 2.79 | 3.91 | 4.23 | 4.63 |
| Sample 2X | 3.36 | 3.52 | 3.66 | 4.17 |
| Sample 3X | 2.83 | 4.22 | 4.30 | 4.77 |
| Comparative Sample 1X | 2.85 | 4.23 | 4.69 | 5.55 |

Comparative Sample 2X had poor solubility in MEK and it was impossible to prepare a film.

**Table 11**

| (Lightfastness test) b* value | | |
|---|---|---|
| | initial stage | after 50 hours |
| Sample 1X | 2.79 | 5.61 |
| Sample 2X | 3.36 | 6.03 |
| Comparative Sample 1X | 2.85 | 6.49 |

Comparative Sample 2X had poor solubility in MEK and it was impossible to prepare a film.

### Experimental Example 4

### Example of near-infrared absorption filter containing diimonium salt compound

0.03% of compound No.6a obtained in the above Example 1 was added to PMMA (polymethyl methacrylate) and injection molding was conducted at 200°C to obtain filters having a thickness of 1 mm and 3 mm. When measured by a spectrophotometer, the filter having a thickness of 1 mm had an average light transmittance of 20% and the filter having a thickness of 3 mm had an average light transmittance 3% at 800-1000 nm, indicating that superior near-infrared absorption filters that effectively absorb near-infrared were obtained.

### Experimental Example 5

### Example of optical recording medium using diimonium salt compound

0. 02 part of compound No.1a obtained in the above Example 1 and 0.10 part of a cyanine dye (OM-57, available from Fuji Photo Film Co., Ltd.) were dissolved in 10 parts of tetrafluoropropanol and the mixture was passed through a 0.2 µm filter to obtain coating liquid. 5 ml of this liquid was dropped on a grooved 5-inch polycarbonate resin substrate by using a pipette, coated by a spin coater and dried to form a recording layer of an organic thin film. The maximum absorption wavelength of the coated film was 719 nm. A gold film was formed on the obtained coated film by a sputtering method as a reflective layer to prepare an optical information recording medium. The obtained optical information recording medium was evaluated using a read/write machine for CD-R and writing and reading were successful. In this way, an optical recording medium superior in processability was obtained and the read/write function was free of problems.

### Experimental Example 6

### Lightfastness test of dye film containing diimonium salt compound

0.3 part of cyanine dye (OM-57) was dissolved in 15 parts of tetrafluoropropanol and 0.04 part of compound No.2a was added to the solution to prepare coating. The obtained coating was spin coated on a polycarbonate substrate to prepare a dye film (Sample 1Y). The obtained dye film was subjected to a lightfastness test using a weatherometer (Ci4000, made by Atlas Electric Devices Co. ) under conditions of light source output: 0.36 W/m², bath temperature: 24°C, black panel temperature: 40°Candhumidity: 30%RH for 50 hours, by applying light from the substrate side. Then the residual ratio of the cyanine dye was measured by a spectrophotometer. The results are shown in Table 12.

Further, dye films were prepared and evaluated in the same manner except that compounds of No. 3a (Sample 2Y) , No. 7a (Sample 3Y) and No.10a (Sample 4Y) , compounds of No. 12a (Sample 5Y) and No.13a (Sample 6Y) and compound No.19a (Sample 7Y) were used instead of compound No.2a, and the results are shown in Table 12.

For comparison, a dye film was prepared and evaluated in the same manner except that hexafluoroantimonate of tetrakis{p-di(n-butyl)aminophenyl}phenylenediimonium (Comparative Sample 1Y) was used instead of compound No.2a. The results are shown in Table 12.

**Table 12**

| (lightfastness test) | | |
|---|---|---|
| | residual ratio of cyanine dye (%) | |
| | initial stage | after 50 hours |
| Sample 1Y | 100 | 72 |
| Sample 2Y | 100 | 82 |
| Sample 3Y | 100 | 73 |
| Sample 4Y | 100 | 80 |
| Sample 5Y | 100 | 78 |
| Sample 6Y | 100 | 75 |
| Sample 7Y | 100 | 78 |
| Comparative Sample 1Y | 100 | 69 |

### Experimental Example 7

### Example of optical recording medium using aminium salt compound

0. 02 part of compound No. 2b obtained in the above Example 7 and 0.10 part of a cyanine dye (OM-57, available from Fuji Photo Film Co., Ltd.) were dissolved in 10 parts of tetrafluoropropanol and the mixture was passed through a 0.2 µm filter to obtain coating liquid. 5 ml of this liquid was dropped on a grooved 5-inch polycarbonate resin substrate by using a pipette, coated by a spin coater and dried to form a recording layer of an organic thin film. The maximum absorption wavelength of the coated film was 719 nm. A gold film was formed on the obtained coated film by a sputtering method as a reflective layer to prepare an optical information recording medium. The obtained optical information recording medium was evaluated using a read/write machine for CD-R and writing and reading were successful. In this way, an optical recording medium superior in processability was obtained and the read/write function was free of problems.

### Experimental Example 8

### Lightfastness test of dye film containing aminium salt compound

0.3 part of cyanine dye (OM-57) was dissolved in 15 parts of tetrafluoropropanol and 0.04 part of compound No.2b was added to the solution to prepare coating. The obtained coating was spin coated on a polycarbonate substrate to prepare a dye film (Sample 1Z). The obtained dye film was subjected to a lightfastness test using a weatherometer (Ci4000, made by Atlas Electric Devices Co. ) under conditions of light source output: 0.36 W/m², bath temperature: 24°C, black panel temperature: 40°C and humidity: 30%RH for 150 hours, by applying light from the substrate side. Then the residual ratio of the cyanine dye was measured by a spectrophotometer. The results are shown in Table 13.

Further, dye films were prepared and evaluated in the same manner except that compounds of No. 3b (Sample 2Z), No. 4b (Sample 3Z) and No. 7b (Sample 4Z) and compound No.12b (Sample 5Z) were used instead of compound No.2b, and the results are shown in Table 13.

For comparison, a dye film was prepared and evaluated in the same manner except that hexafluoroantimonate of tetrakis{p-di(n-butyl)aminophenyl)phenylenediimonium (Comparative Sample 1Z) was used instead of compound No.2b. The results are shown in Table 13.

**Table 13**

| (lightfastness test) | | |
|---|---|---|
| | residual ratio of cyanine dye (%) | |
| | initial stage | after 150 hours |
| Sample 1Z | 100 | 48 |
| Sample 2Z | 100 | 51 |
| Sample 3Z | 100 | 53 |
| Sample 4Z | 100 | 32 |
| Sample 5Z | 100 | 59 |
| Comparative Sample 3Z | 100 | 29 |

## Claims

1. A mixture comprising two or more oxides obtained by oxidizing two or more compounds represented by the formula (1): 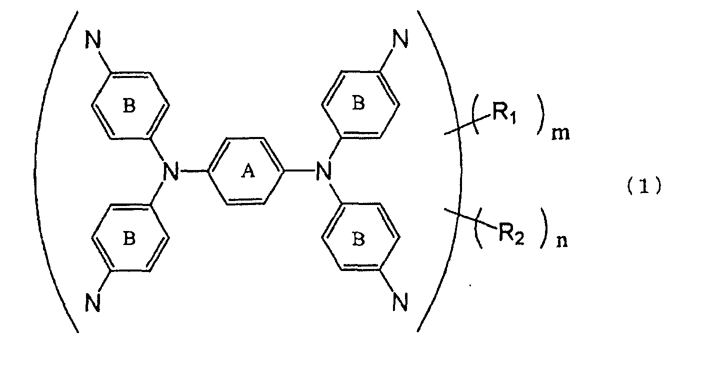 wherein ring A and ring B optionally have substituents, R₁ and R₂ attached to terminal nitrogen atoms of a tetrakis(aminophenyl)phenylenediamine skeleton are different from each other and each being a substituted or unsubstituted alkyl group having 1 to 8 carbon atoms, a cycloalkyl group, an alkenyl group or an aryl group, and m and n are integers of 0 to 8 satisfying the relationship: m+n=8.

2. Amixture comprising two or more diimonium salt compounds obtained by oxidizing two or more compounds represented by the formula (1): 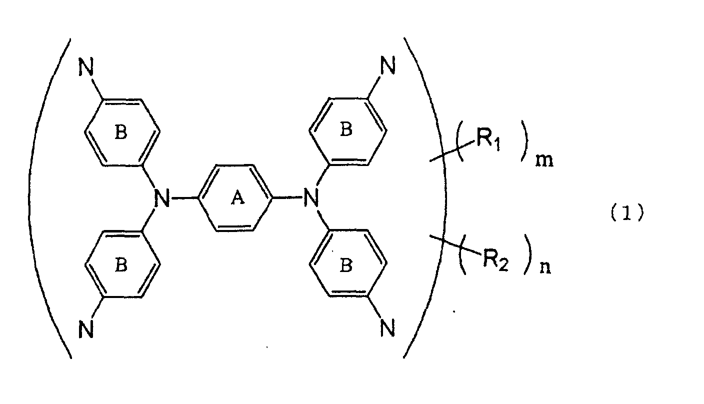 wherein ring A and ring B optionally have substituents, R₁ and R₂ attached to terminal nitrogen atoms of a tetrakis(aminophenyl)phenylenediamine skeleton are different from each other and each being a substituted or unsubstituted alkyl group having 1 to 8 carbon atoms, a cycloalkyl group, an alkenyl group or an aryl group, and m and n are integers of 0 to 8 satisfying the relationship: m+n=8.

3. The mixture of diimonium salt compounds of claim 2, wherein the ring A and the ring B have no substituent or have, as substituents, at least one selected from the group consisting of a halogen atom (s) , an alkyl group (s) and a cyano group(s).

4. The mixture of diimonium salt compounds of claim 3, wherein R₁ and R₂ are a substituted or unsubstituted alkyl group(s) or an alkenyl group(s).

5. The mixture of diimonium salt compounds of claim 4, wherein at least one of R₁ and R₂ is a cyano (C1-C6) alkyl group.

6. The mixture of diimonium salt compounds of claim 5, wherein the cyano(C1-C6)alkyl group is a cyanopropyl group.

7. A near-infrared absorption filter comprising the mixture of diimonium salt compounds of any one of claims 2 to 6.

8. A near-infrared absorption composition obtained by adding the mixture of diimonium salt compounds of any one of claims 2 to 6 to a resin(s).

9. An optical information recording medium containing, in a recording layer, the mixture of diimonium salt compounds of any one of claims 2 to 6.

10. A mixture comprising two or more aminium salt compounds obtained by oxidizing two or more compounds represented by the formula (1): 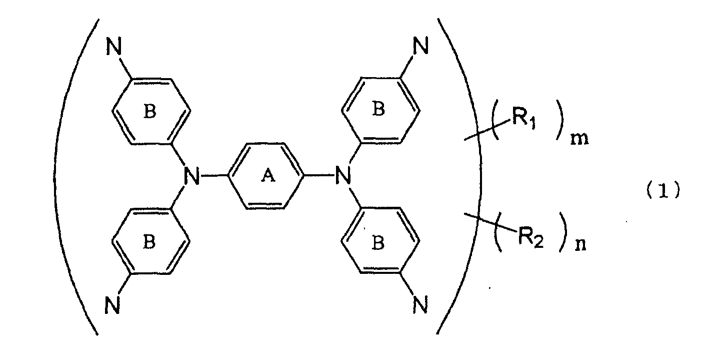 wherein ring A and ring B optionally have substituents, R₁ and R₂ attached to terminal nitrogen atoms of a tetrakis(aminophenyl)phenylenediamine skeleton are different from each other and each being a substituted or unsubstituted alkyl group having 1 to 8 carbon atoms, a cycloalkyl group, an alkenyl group or an aryl group, and m and n are integers of 0 to 8 satisfying the relationship: m+n=8.

11. The mixture of aminium salt compounds of claim 10, wherein the ring A and the ring B have no substituent or have, as substituents, at least one selected from the group consisting of a halogen atom (s), an alkyl group (s) and a cyano group (s).

12. The mixture of aminium salt compounds of claim 11, wherein R₁ and R₂ are a substituted or unsubstituted alkyl group(s) or an alkenyl group(s).

13. The mixture of aminium salt compounds of claim 12, wherein at least one of R₁ and R₂ is a cyano(C1-C6)alkyl group.

14. The mixture of aminium salt compounds of claim 13, wherein the cyano(C1-C6)alkyl group is a cyanopropyl group.

15. A near-infrared absorption filter comprising the mixture of aminium salt compounds of any one of claims 10 to 14.

16. A near-infrared absorption composition obtained by adding the mixture of diimonium salt compounds of any one of claims 10 to 14 to a resin(s).

17. An optical information recording medium containing, in a recording layer, the mixture of aminium salt compounds of any one of claims 10 to 14.

18. Amixture of two or more oxide precursors which can produce the mixture of diimonium salt compounds of any one of claims 2 to 6 or the mixture of aminium salt compounds of any one of claims 10 to 14.

19. A mixture of diimonium salt compounds containing two or more compounds represented by the formula (2): 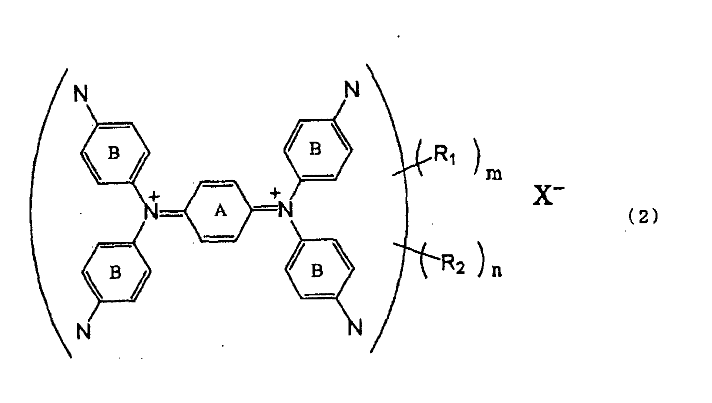 wherein ring A and ring B optionally have substituents, R₁ and R₂ attached to terminal nitrogen atoms of a tetrakis(aminophenyl)phenylenediamine skeleton are different from each other and each being a substituted or unsubstituted alkyl group having 1 to 8 carbon atoms, a cycloalkyl group, an alkenyl group or an aryl group, m and n are integers of 0 to 8 satisfying the relationship: m+n=8 and X is an atomic group having a negative electric charge(s) necessary for neutralizing a positive electric charge(s).

20. A mixture of aminium salt compounds containing two or more compounds represented by the formula (3): 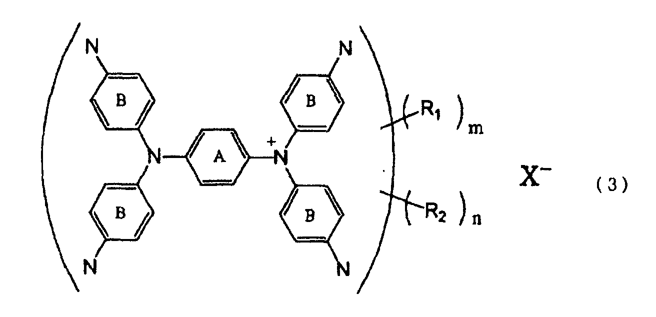 wherein ring A and ring B optionally have substituents, R₁ and R₂ attached to terminal nitrogen atoms of a tetrakis(aminophenyl)phenylenediamine skeleton are different from each other and each being a substituted or unsubstituted alkyl group having 1 to 8 carbon atoms, a cycloalkyl group, an alkenyl group or an aryl group, m and n are integers of 0 to 8 satisfying the relationship: m+n=8 and X is an atomic group having a negative electric charge(s) necessary for neutralizing a positive electric charge(s).
